# FASCICULE DE BREVET EUROPEEN

(11) **EP 0 680 930 B1**
(45) Date de publication et mention de la délivrance du brevet: **28.10.1998**
(21) Numéro de dépôt: 95400860.3
(22) Date de dépôt: 18.04.1995
(51) Int. Cl.: C01F 17/00, C08K 3/30, C09D 7/12

(54) **Composition à base d'un sulfure de terre rare comprenant au moins un élément alcalin, son procédé de préparation et son utilisation comme pigment coloré**
Zusammensetzung auf der Basis von Sulfid von seltener Erde enthaltend zumindest ein Alkalimetallelement, Verfahren zu seiner Herstellung und seine Verwendung als gefärbtes Pigment
Rare earth sulphide composition containing at least one alkali metal element, process for its preparation and its use as colored pigment

(30) Priorité: 06.05.1994 FR 9405588
(43) Date de publication de la demande: 08.11.1995
(73) Titulaire: RHODIA CHIMIE, 92408 Courbevoie Cédex (FR)
(72) Inventeur: Macaudiere, Pierre, F-92600 Asnières-sur-Seine (FR)
(74) Mandataire: Dubruc, Philippe

(56) Documents cités:
- EP-A- 0 545 746
- DE-A- 2 126 190
- US-A- 3 748 095
- US-A- 4 619 792
- MATERIALS RESEARCH BULLETIN, vol. 19, no. 9, 1984 USA, pages 1215-1220, G. ADACHI ET AL 'preparation and structure of sodium rare-earth sulfides'

## Description

La présente invention concerne une composition à base d'un sulfure de terre rare comprenant au moins un élément alcalin, son procédé de préparation et son utilisation comme pigment coloré.

Les pigments minéraux de coloration sont déjà largement utilisés dans de nombreuses industries notamment dans celles des peintures, des matières plastiques et des céramiques. Dans de telles applications, les propriétés que sont, entre autres, la stabilité thermique et/ou chimique, la dispersabilité (aptitude du produit à se disperser correctement dans un milieu donné), la couleur intrinsèque, le pouvoir de coloration et le pouvoir opacifiant, constituent autant de critères particulièrement importants à prendre en considération dans le choix d'un pigment convenable.

Malheureusement, le problème est que la plupart des pigments minéraux qui conviennent pour des applications telles que ci-dessus et qui sont effectivement utilisés à ce jour à l'échelle industrielle, font généralement appel à des métaux (cadmium, plomb, chrome, cobalt notamment) dont l'emploi devient de plus en plus sévèrement réglementé, voire interdit, par les législations de nombreux pays, compte tenu en effet de leur toxicité réputée très élevée. On peut ainsi plus particulièrement citer, à titre d'exemples non limitatifs, le cas des pigments rouges à base de séléniure de cadmium et/ou de sulfoséléniure de cadmium, et pour lesquels des substituts à base de sulfures de terres rares ont déjà été proposés par la Demanderesse. Des compositions à base de sesquisulfures de terre rare et d'éléments alcalins ont ainsi été décrits dans EP-A-545746. Ces compositions, qui sont obtenues par un procédé consistant essentiellement à chauffer un mélange à base d'un composé de terre rare, d'un élément alcalin et du soufre, se sont avérées être des substituts particulièrement intéressants.

Toutefois, le besoin s'est fait sentir de disposer de produits à qualités pigmentaires encore améliorées et qui puissent être obtenus avec un procédé encore plus simple industriellement.

L'objet de la présente invention est de fournir de tels produits et un tel procédé.

Dans ce but, la composition selon l'invention, à base d'un sulfure de terre rare comprenant au moins un élément alcalin est caractérisée en ce que le sulfure est constitué de grains monocristallins entiers de taille moyenne d'au plus 1,5µm.

D'autre part, le procédé de préparation, selon l'invention, d'une composition à base d'un sulfure de terre rare comprenant au moins un élément alcalin est caractérisé en ce qu'on met en présence au moins un carbonate ou un hydroxycarbonate de terre rare avec au moins un composé d'un élément alcalin et on les chauffe en présence d'au moins un gaz choisi parmi le sulfure d'hydrogène ou le sulfure de carbone.

Les produits obtenus présentent une granulométrie particulièrement fine, notamment inférieure à 2µm et de très grandes qualités de couleur. Le procédé de préparation est d'une mise en oeuvre simple et il présente en outre l'avantage de permettre de travailler à des températures relativement basses par exemple dès 500°C

D'autres caractéristiques, détails et avantages de l'invention apparaîtront encore plus complètement à la lecture de la description qui va suivre, ainsi que des divers exemples concrets mais non limitatifs destinés à l'illustrer.

La composition de l'invention va tout d'abord être décrite.

Celle-ci est à base essentiellement d'un sulfure de terre rare. Ce sulfure de terre rare peut être tout particulièrement un sesquisulfure.

Par terre rare on entend ici les éléments du groupe constitué par l'yttrium et les éléments de la classification périodique de numéro atomique compris inclusivement entre 57 et 71. La classification périodique des éléments à laquelle il est fait référence est celle publiée dans le Supplément au Bulletin de la Société Chimique de France n° 1 (janvier 1966). On doit entendre ici aussi que le sulfure ou le sesquisulfure de la composition de l'invention peut comprendre plusieurs terres rares, tout ce qui est donc décrit par la suite en référence à une terre rare s'applique aussi au cas où plusieurs terres rares sont présentes.

L'invention s'applique en particulier aux sesquisulfures pour lesquels la terre rare est le cérium ou le lanthane.

Le sulfure ou le sesquisulfure de la composition de l'invention comprend en outre au moins un élément alcalin (élément dopant). Cet élément alcalin peut être choisi notamment parmi le lithium, le sodium ou le potassium. Le sodium peut être tout particulièrement choisi. Bien entendu, le sulfure ou sesquisulfure de la composition de l'invention peut comprendre plusieurs éléments alcalins et, là aussi, tout ce qui est donc décrit par la suite en référence à un alcalin s'applique aussi au cas où plusieurs alcalins sont présents.

Selon un mode de réalisation préféré de l'invention, cet élément alcalin est inclus au moins en partie dans le réseau cristallin du sulfure ou sesquisulfure . Selon une variante de ce mode de réalisation, l'élément alcalin est inclus essentiellement ou totalement dans le réseau cristallin.

Le sesquisulfure de la composition de l'invention peut posséder notamment une structure cristallographique cubique de type Th₃P₄, qui présente des lacunes au niveau du réseau des cations; cette structure lacunaire peut être symbolisée en donnant aux sesquisulfures la formule M_{10,66} [ ]_{1,33} S₁₆ (voir notamment à ce sujet, W.H. ZACHARIASEN, "Crystal Chemical Studies of the 5f-Series of Elements. The Ce₂S₃-Ce₃S₄ Type of Structure", Acta Cryst., (1949), 2, 57).

Selon l'invention, un ou des éléments alcalins peuvent être introduits dans ces lacunes cationiques, jusqu'à saturation ou non de ces dernières. La présence de cet élément au sein du sulfure ou sesquisulfure peut être mise en évidence par simple analyse chimique. Par ailleurs, les analyses en diffraction X montrent qu'il y a conservation de la phase cristalline en Th₃P₄ du sesquisulfure, avec dans certains cas, une modification plus ou moins importante des paramètres de maille, fonction à la fois de la nature et de la quantité de l'élément dopant introduit.

Généralement, la quantité d'élément alcalin est d'au plus 50% de la quantité molaire en terre rare du sulfure ou du sesquisulfure.

Selon une autre caractéristique préférée, la quantité molaire en alcalin est au moins égale à 0,1%, et avantageusement comprise entre 5% et 50% et plus particulièrement 5 et 20%, de la quantité molaire en terre rare.

Une caractéristique essentielle de la composition de l'invention réside dans le fait qu'elle est à base d'un sulfure constitué de grains monocristallins entiers de taille moyenne d'au plus 1,5 micron, plus particulièrement d'au plus 1 micron. Par grain entier, on entend un grain qui n'a pas été rompu ou brisé. Des grains peuvent en effet être brisés ou rompus lors d'un broyage. Les photos en microscopie électronique à balayage du produit de l'invention permettent de montrer que les grains qui le constituent n'ont pas été brisés. Il faut aussi noter que le sulfure ou le sesquisulfure de la composition de l'invention est désagglomérable, c'est à dire que s'il ne se présente pas directement sous forme de grains monocristallins entiers, il peut se présenter sous forme d'agglomérats de particules pouvant être constituées de grains agglomérés et/ou légèrement frittés qui peuvent donner par désagglomération dans des conditions douces les grains monocristallins entiers. La combinaison du caractère entier du grain et de sa faible taille est probablement à l'origine des très bonnes qualités pigmentaires de la composition de l'invention.

En ce qui concerne plus précisément la granulométrie du produit de l'invention, celui-ci présente une taille moyenne généralement inférieure à 2µm, plus particulièrement comprise entre 0,7 et 1,5 µm. Après une désagglomération dans des conditions douces, la taille moyenne peut être d'au plus 1,5µm et avantageusement comprise entre 0,3 et 0,8µm. La taille de particules est mesurée par granulométrie CILAS.

Les compositions à base de sulfures ou de sesquisulfures de l'invention peuvent présenter une très large gamme de couleurs en fonction notamment de la terre rare et de l'élément alcalin qui rentrent dans leur composition, ce qui implique que leurs coordonnées chromatiques peuvent varier dans une très large fourchette. Des exemples vont être donnés ci-dessous.
- les sulfures de cérium ont une couleur variant du brun au rouge selon les conditions de préparation, en particulier la température de calcination. Ils sont bruns ou rouges sang selon que l'on ait la phase Ce₂S₃ β orthorhombique (J.C.P.D.S. 20 269) ou la phase Ce₂S₃ γ cubique (J.C.P.D.S. 27 104);
- avec le lanthane, on obtient des produits jaunes, et ceci avec une structure La₂S₃ cubique (J.C.P.D.S. 25 1041);
- une coloration verte peut être obtenue avec le néodyme, et une coloration vert-jaune avec le praséodyme. Les produits présentent alors respectivement la structure Nd₂S₃ cubique (J.C.P.D.S. 26 1450) et la structure Pr₂S₃ cubique (J.C.P.D.S. 27 481);
- on dispose d'un produit jaune-marron avec le dysprosium de structure Dy₂S₃ cubique (J.C.P.D.S. 26 594);
- des produits présentant différentes nuances de marron peuvent aussi être obtenus : ocre avec le terbium de structure Tb₂S₃ cubique, brun avec l'erbium de structure Er₂S₃ monoclinique (J.C.P.D.S. 21 324) et beige foncé avec l'yttrium de structure Y₂S₃ monoclinique (J.C.P.D.S. 22 996);
- d'autres exemples de couleurs que l'on peut obtenir sont enfin : brun-gris avec le samarium de structure Sm₂S₃ cubique (J.C.P.D.S. 26 1480), brun-vert avec le gadolinium de structure Gd₂S₃ γ cubique (J.C.P.D.S. 26 1424), vert-or avec le thullium de structure Tm₂S₃ monoclinique (J.C.P.D.S. 30 1364).

Le procédé de préparation des compositions de l'invention va maintenant être décrit.

Une première caractéristique de ce procédé réside dans la nature des produits de départ. La terre rare est apportée sous forme d'un carbonate ou d'un hydroxycarbonate.

Il est avantageux d'utiliser un carbonate ou hydroxycarbonate de fine granulométrie, notamment de taille moyenne d'au plus 1µm.

En ce qui concerne l'élément alcalin, celui-ci peut être apporté sous différentes formes. On peut mentionner par exemple les sels de ces éléments. Toutefois, il est avantageux d'utiliser un carbonate alcalin.

De préférence, on forme une poudre à base d'un mélange homogène du carbonate ou de l'hydroxycarbonate de terre rare avec le composé d'un élément alcalin.

Selon une variante particulière de l'invention, on utilise un carbonate ou un hydroxycarbonate de terre rare préalablement imprégné d'un élément alcalin. On forme dans ce cas une solution aqueuse d'un sel ou d'un hydroxyde alcalin et on imprègne le carbonate ou l'hydroxycarbonate de terre rare avec la solution puis on sèche, selon toute méthode permettant de limiter la formation d'oxyde par exemple : étuve, atomisation...

Une autre caractéristique du procédé de l'invention est la nature du gaz sulfurant. Ce gaz peut être le sulfure d'hydrogène ou le sulfure de carbone. Selon un mode de réalisation préféré de l'invention, on utilise un mélange de ces deux gaz. L'utilisation d'un tel mélange favorise l'obtention de produits phasiquement purs et limite le dépôt de carbone sur le produit obtenu. Le gaz ou le mélange de gaz sulfurant peut être mis en oeuvre avec un gaz inerte comme l'argon ou l'azote.

Le chauffage se fait à une température qui peut être aussi faible que 500°C. On a pu observer en effet la formation des produits recherchés dès cette température. Il s'agit là d'un avantage important vis à vis des procédés connus qui nécessitent des températures élevées, généralement d'au moins 900°C. Habituellement, on met en oeuvre le procédé de l'invention à une température comprise entre 500 et 900°C, les températures élevées favorisant l'obtention de produits phasiquement purs.

La durée du chauffage correspond au temps nécessaire pour l'obtention du sulfure ou du sesquisulfure désiré et cette durée est d'autant plus courte que la température est élevée. A titre d'exemple, cette durée peut aller de deux heures environ pour une température de 500°C à quinze minutes environ pour une température de 800°C.

La réaction se fait généralement avec une pression partielle du sulfure d'hydrogène et/ou du sulfure de carbone qui est comprise entre 0,1 et 1.10⁵Pa.

Enfin, le procédé peut être mis en oeuvre dans un réacteur ouvert.

Le produit obtenu à l'issue du chauffage présente habituellement une taille moyenne inférieure à 2µm, plus particulièrement inférieure à 1,5 µm. Cependant, si on souhaite obtenir une granulométrie plus fine, le produit peut être désaggloméré. Comme on l'a mentionné précédemment, une désagglomération dans des conditions douces, par exemple un broyage du type jet d'air, est suffisant pour obtenir une taille moyenne qui peut être inférieure à 1,5µm et par exemple d'au plus 1µm et avantageusement comprise entre 0,3 et 0,8µm.

L'invention concerne aussi les pigments colorés comprenant une composition à base d'au moins un sulfure ou sesquisulfure, du type ci-dessus ou obtenu par le procédé décrit précédemment.

Les compositions à base de sulfure ou de sesquisulfure ou les pigments selon l'invention possèdent un très bon pouvoir de coloration et un très bon pouvoir couvrant et, de ce fait, conviennent parfaitement à la coloration de nombreux matériaux, tels que plastiques, peintures et autres.

Ainsi, et plus précisément encore, ils peuvent être utilisés dans la coloration de matières plastiques qui peuvent être du type thermoplastiques ou thermodurcissables.

Comme résines thermoplastiques susceptibles d'être colorées selon l'invention, on peut citer, à titre purement illustratif, le chlorure de polyvinyle, l'alcool polyvinylique, le polystyrène, les copolymères styrène-butadiène, styrène-acrylonitrile, acrylonitrilebutadiène-styrène (A.B.S.), les polymères acryliques notamment le polyméthacrylate de méthyle, les polyoléfines telles que le polyéthylène, le polypropylène, le polybutène, le polyméthylpentène, les dérivés cellulosiques tels que par exemple l'acétate de cellulose, l'acéto-butyrate de cellulose, l'éthylcéllulose, les polyamides dont le polyamide 6-6.

Concernant les résines thermodurcissables pour lesquelles les compositions ou les pigments selon l'invention conviennent également, on peut citer, par exemple, les phénoplastes, les aminoplastes notamment les copolymères urée-formol, mélamine-formol, les résines époxy et les polyesters thermodurcissables.

On peut également mettre en oeuvre les compositions ou les pigments de l'invention dans des polymères spéciaux tels que des polymères fluorés en particulier le polytétrafluoréthylène (P.T.F.E.), les polycarbonates, les élastomères silicones, les polyimides.

Dans cette application spécifique pour la coloration des plastiques, on peut mettre en oeuvre les compositions ou les pigments de l'invention directement sous forme de poudres. On peut également, de préférence, les mettre en oeuvre sous une forme pré dispersée, par exemple en prémélange avec une partie de la résine, sous forme d'un concentré pâte ou d'un liquide ce qui permet de les introduire à n'importe quel stade de la fabrication de la résine.

Ainsi, les compositions ou les pigments selon l'invention peuvent être incorporés dans des matières plastiques telles que celles mentionnées ci-avant dans une proportion pondérale allant généralement soit de 0,01 à 5% (ramenée au produit final) soit de 40 à 70% dans le cas d'un concentré.

Les compositions ou les pigments de l'invention peuvent être également utilisés dans le domaine des peintures et lasures et plus particulièrement dans les résines suivantes : résines alkydes dont la plus courante est dénommée glycérophtalique; les résines modifiées à l'huile longue ou courte; les résines acryliques dérivées des esters de l'acide acrylique (méthylique ou éthylique) et méthacrylique éventuellement copolymérisés avec l'acrylate d'éthyle, d'éthyl-2 hexyle ou de butyle; les résines vinyliques comme par exemple l'acétate de polyvinyle, le chlorure de polyvinyle, le butyralpolyvinylique, le formalpolyvinylique, et les copolymères chlorure de vinyle et acétate de vinyle ou chlorure de vinylidène; les résines aminoplastes ou phénoliques le plus souvent modifiées; les résines polyesters; les résines polyuréthannes; les résines époxy; les résines silicones.

Généralement, les compositions ou les pigments sont mis en oeuvre à raison de 5 à 30% en poids de la peinture, et de 0,1 à 5% en poids du lasure.

Enfin, les compositions ou les pigments selon l'invention sont également susceptibles de convenir pour des applications dans l'industrie du caoutchouc, notamment dans les revêtements pour sols, dans l'industrie du papier et des encres d'imprimerie, dans le domaine de la cosmétique, ainsi que nombreuses autres utilisations comme par exemple, et non limitativement, le finissage des cuirs et les revêtements stratifiés pour cuisines et autres plans de travail, les céramiques.

En ce qui concerne plus particulièrement la cosmétique, les compositions ou pigments de l'invention peuvent être utilisés dans les vernis à ongles et dans les fards tels que rouges à lèvres, fards secs, fards gras ou fonds de teint.

Ils peuvent donc être mises en oeuvre dans les vernis à ongles qui contiennent généralement:
- un agent filmogène à base de nitrocellulose,
- une résine, résine dammer naturelle ou résine synthétique du type formaldéhyde-sulfamide, résine polystyrène, résine polyvinylique etc...,
- un plastifiant par exemple, le phtalate de diéthyle, le phtalate de dibutyle, le phtalate de dioctyle, le tricrésylphosphate, le stéarate de n-butyle, le diacétate de résorcine ou leur mélange,
- un dissolvant tel que l'alcool éthylique, isopropylique, butylique, isobutylique, l'acétate d'éthyle, l'acétate de butyle ou le plus souvent le mélange de ces solvants,
- un diluant, notamment le toluène ou le xylène,
- éventuellement d'autres additifs, parfum ou produit nacrant (flocons de mica enrobés d'oxychlorure de bismuth ou de bioxyde de titane).

On donne ci-après un exemple de composition-type:
- de 10 à 15% en poids de nitrocellulose,
- de 10 à 15% en poids de résine,
- de 3 à 5% en poids de plastifiant(s),
- de 3 à 5% en poids de pigment(s),
- q.s.p. 100% en poids de solvant(s).

Généralement, les compositions ou pigments sont broyés dans une masse plastique constituée de nitrocellulose et de plastifiant(s) qui est ensuite mise en solution dans le(s) solvant(s).

Une autre application des compositions ou pigments de l'invention est celle des rouges à lèvres.

Les compositions ou pigments sont mis en oeuvre le plus souvent à raison d'une concentration pondérale de 5 à 15% exprimée par rapport à la formulation totale qui renferme :
- un excipient formé d'un mélange de divers corps pour assurer la consistance: cire d'abeille, cire de carnauba, ozocérites, paraffine, cires synthétiques ou leur mélange et d'un excipient mou permettant d'ajuster la consistance tel que le beurre de cacao, la vaseline, les huiles blanches hydrogénées par exemple, l'huile de palme, d'arachide ou de ricin,
- divers additifs notamment un parfum ou arome et le myristate d'isopropyle ou le palmitate d'isopropyle qui donne du glissant,
- un solvant intermédiaire pour mettre en suspension le pigment dans la phase lipophile qui peut être l'huile de ricin ou un glycol comme le polyoxyéthylèneglycol 400 ou des esters d'acides gras : monoricinoléate de propylène glycol, myristate d'isopropyle, palmitate d'isopropyle, stéarate de butyle.

Les fards à yeux et les fards à joues peuvent se présenter sous forme de fards secs ou de fards gras. La teneur en compositions ou pigments dans de tels fards peut varier dans de larges limites de 5 à 20%.

Les fards secs sont des poudres (talc, carbonate de magnésium, stéarate de zinc) qui sont chargées en pigments et agglomérées soit avec de la méthylcellulose, soit avec des stéarates.

On donne, à titre d'exemple la composition d'un fard à paupières:

| | |
|---|---|
| - silicate d'aluminium et de magnésium (Veegum F) | 7% en poids |
| - talc | 50% en poids |
| - oxyde de zinc | 4% en poids |
| - stéarate de zinc | 11% en poids |
| - kaolin | 10% en poids |
| - pigment | 18% en poids |

Les compositions ou pigments de l'invention peuvent également être employés dans les formulations de fonds de teint.

Les fonds de teint se présentent sous forme d'émulsion en général du type huile dans l'eau.

La phase lipophile comprend le plus souvent:
- un composant huileux tel que l'huile de vaseline, des esters d'acides gras et d'alcools éventuellement gras, par exemple, l'oléate d'oléyle, l'oléate de décyle, le stéarate d'octyle, l'adipate de di-n-butyle, le myristate d'isopropyle, le palmitate d'isopropyle, le stéarate d'isopropyle, les esters d'acides caprique et caprylique d'alcools gras saturés ayant de 12 à 18 atomes de carbone, une huile de silicone ou leur mélange entre eux,
- un agent émulsifiant du type anionique et/ou non-ionique et plus précisément les sels d'acides gras, stéarate de sodium, de potassium ou d'ammonium, palmitate de sodium les esters de sorbitan et d'acides gras tels que, par exemple, l'acide laurique, l'acide palmitique, l'acide stéarique; les esters polyoxyéthylénés de sorbitan et d'acides gras contenant de 4 à 20 moles d'oxyde d'éthylène par mole d'ester les alcools gras polyoxyéthylénés contenant de 2 à 23 moles d'oxyde d'éthylène par mole d'alcool, ledit alcool pouvant être notamment l'alcool laurique, l'alcool cétylique, l'alcool stéarylique, l'alcool oléylique; le mono-et distéarate de glycérol, le mono-et dioléate de glycérol; les acides gras polyloxyéthylénés et en particulier le stéarate polyoxyéthyléné contenant de 18 à 100 moles d'oxyde d'éthylène par mole d'acide,
- un agent permettant d'ajuster la consistance qui peut être un alcool gras ou un acide gras et plus précisément l'alcool cétylique, l'alcool stéarylique, l'acide stéarique.

Quant à la phase hydrophile, elle est constituée d'eau, de préférence distillée et de divers additifs, notamment:
- un agent humectant qui peut être, par exemple, le propylèneglycol, le glycérol, le sorbitol,
- un agent conservateur et plus particulièrement l'ophénylphénol et les acides suivants, leurs sels (Na, K, NH₄) ou leurs esters ayant de 1 à 4 atomes de carbone l'acide benzoïque, l'acide salicylique, l'acide sorbique, l'acide p-hydroxybenzoïque,
- un agent de stabilité notamment les dérivés cellulosiques dont la carboxyméthylcellulose et la gomme Xanthane.

Une illustration d'une formulation pour fonds de teint est donnée ci-après:
- phase lipophile:

| | |
|---|---|
| - huile de vaseline | 15% en poids |
| - mono-et distéarate de glycérol | 4% en poids |
| - alcool cétylique | 1% en poids |

- phase hydrophile:

| | |
|---|---|
| - eau distillée q.s.p. | 100% en poids |
| - propylène glycol | 3% en poids |
| - para-oxybenzoate de méthyle | 0,05% en poids |
| - para-oxybenzoate de propyle | 0,1% en poids |
| - pigment coloré | 1 à 10% en poids |
| - oxyde de titane | 3% en poids |

La préparation des formulations de fonds de teint est conduite en dispersant d'abord le pigment dans la phase lipophile maintenue vers 60-80°C puis la phase hydrophile maintenue à une des températures comprises dans l'intervalle précité est ajoutée sous agitation et lentement dans la phase lipophile.

Dans l'exposé qui précède, il est donné des exemples de formulations destinées à la cosmétique dans lesquelles peuvent convenir les compositions ou pigments de l'invention et il va sans dire que ces exemples de même que les différents composants cités ne présentent aucun caractère limitatif et ne sont donnés qu'à titre illustratif.

L'invention concerne aussi les compositions de matière colorées notamment du type plastiques, peintures, lasures, caoutchoucs, céramiques, glaçures, papiers, encres, produits cosmétiques, teintures et revêtements stratifiés, caractérisées en ce qu'elles comprennent une composition ou un pigment coloré du type décrit ci-dessus.

Des exemples concrets mais non limitatifs vont maintenant être donnés.

Dans ces exemples, les coordonnées chromatiques L*, a* et b* sont données ici et pour le reste de la description dans le système CIE 1976 (L*, a*, b*) tel que défini par la Commission Internationale d'Eclairage et répertorié dans le Recueil des Normes Françaises (AFNOR), couleur colorimétrique n° X08-12 (1983). Elles sont déterminées au moyen d'un colorimètre commercialisé par la Société Pacific Scientific. La nature de l'illuminant est D65. La surface d'observation est une pastille circulaire de 12,5 cm² de surface. Les conditions d'observations correspondent à une vision sous un angle d'ouverture de 10°. Dans les mesures données, la composante spéculaire est exclue.
L* donne une mesure de la réflectance (nuance clair/sombre) et varie ainsi de 100 (blanc) à 0 (noir).
a* et b* sont les valeurs des tendances colorées:
   a* positif = rouge
   a* négatif = vert
   b* positif = jaune
   b* négatif = bleu
L* représente donc la variation du noir au blanc, a* la variation du vert au rouge et b* la variation du jaune au bleu.

### EXEMPLE 1

Cet exemple concerne la préparation d'un sesquisulfure de cérium cubique comprenant du sodium.

Dans un mortier, on introduit 6g d'hydroxycarbonate de cérium de granulométrie CILAS inférieure à 1µm et 0,22g de carbonate de sodium anhydre. Le rapport molaire Na/Ce est alors de 0,2.

L'ensemble est alors broyé de façon à obtenir un mélange homogène. Ce mélange est ensuite porté à 500°C pendant 2 heures sous un balayage continu d'un mélange gazeux contenant de l'argon, du sulfure d'hydrogène et du sulfure de carbone (50%, 20%, 30% respectivement en volume).

Le produit ainsi obtenu se révèle être principalement du sesquisulfure de cérium cubique, par analyse en diffraction X. Sa granulométrie est inférieure à 1 µm.

### EXEMPLE 2

Un sesquisulfure de cérium cubique dopé au lithium a été préparé en calcinant à 800°C durant 90 minutes en temps de palier sous une atmosphère contenant du sulfure de carbone à raison de 0,3.10⁵Pa en pression partielle et du sulfure d'hydrogène à raison de 0,2.10⁵Pa dans de l'argon, un mélange d'hydroxycarbonate de cérium du même type que celui de l'exemple 1 et de carbonate de lithium. Le rapport molaire Li/Ce dans le mélange a été fixé à 0,10.

Le produit obtenu est un pigment rouge dont les coordonnées couleur et la granulométrie sont les suivantes:
L*= 45,1
a* = 46,1
b* = 34,1
D₅₀= 1,5µm

Après simple désagglomération, le produit obtenu se présente sous forme de grains monocristallins entiers de taille inférieure au micron. Le D₅₀ du produit est de 0,65µm.

### EXEMPLE 3

A l'inverse des exemples ci-dessus, l'alcalin est réparti uniformément dans le précurseur de terre rare par imprégnation de ce dernier par le sel alcalin (carbonate de sodium) en solution. L'alcalin est ici présent dans un rapport Na/Ce de 0,15. Après traitement par un mélange sulfurant contenant 30% (volumique) de CS₂, 20% de sulfure d'hydrogène et le complément en argon, et à 800°C en 30 minutes de temps de palier, le sesquisulfure est cristallisé dans la structure Th₃P₄.

Les caractéristiques couleur sont les suivantes:
L*= 55,9
a* = 50,3
b* = 43,3
D₅₀ = 1µm

Après désagglomération, le produit obtenu présente un D₅₀ de 0,65µm ainsi que des grains monocristallins entiers de taille inférieure au micron.

### EXEMPLE 4

L'exemple suivant décrit la synthèse d'un sulfure de cérium de très faible granulométrie. Sous une pression partielle de 0,3.10⁵Pa de CS₂, de 0,2.10⁵Pa de H₂S et le complément en argon, un carbonate de cérium de granulométrie CILAS de 0,7µm est transformé en présence de carbonate de sodium dans un rapport Na/Ce de 0,15 en sulfure de cérium cubique, à une température de 800°C durant 30 mn en palier. Il présente les caractéristiques couleur et de granulométrie suivantes:
L*= 55,6
a* = 46,2
b* = 45,1
D₅₀ = 0,9µm

Après simple désagglomération, le diamètre moyen est de 0,55µm. Le produit se présente sous forme de grains monocristallins entiers de taille inférieure au micron.

### EXEMPLE 5

A l'inverse des exemples ci-dessus, un mélange contenant uniquement du sulfure de carbone comme agent sulfurant, dilué à 30% dans de l'argon est utilisé. On mélange 6 g d'un hydroxycarbonate de cérium à 0,29 g de carbonate de sodium et le mélange est porté à 800°C durant 5 h. On obtient un produit qui peut être divisé en deux fractions selon un critère de couleur. Les analyses RX montrent en effet une phase cubique pure pour une fraction rouge et une phase cubique ainsi qu'une phase oxysulfure brune pour la seconde fraction

Les coordonnées couleur de la fraction la plus rouge sont les suivantes:
L*= 49,8
a* = 48,8
b* = 40,2
D₅₀ = 2,0µm

Après désagglomération, le diamètre moyen est de 1,45µm.
La sulfuration en présence de sulfure de carbone et de sulfure d'hydrogène (30 et 20% en volume, le complément en argon) de ce même hydroxycarbonate de cérium aboutit à une phase unique de sesquisulfure de cérium cubique dont les coordonnées chromatiques sont les suivantes:
L*= 50,7
a* = 51,5
b* = 42,1

Le produit se présente sous forme de grains monocristallins entiers de taille inférieure au micron.

### EXEMPLE 6

Un hydroxycarbonate de cérium de granulométrie inférieure à 1 µm est mélangé à un carbonate de sodium à raison d'un rapport Na/Ce = 0,15. Ce mélange porté à 750°C en palier de 2 h, sous une atmosphère sulfurante de CS₂, H₂S et argon (30%, 20% et 50% respectivement en volume), conduit à un sulfure de cérium de type Th₃P₄ dont les coordonnées couleur sont les suivantes:
L*= 52,2
a* = 50,4
b* = 46,7
D₅₀ = 1,4µm

Après désagglomération, le diamètre moyen est de 0,8µm. Le produit se présente sous forme de grains monocristallins entiers de taille inférieure au micron.

### EXEMPLE 7

On réalise ici la préparation d'un pigment avec un cycle thermique très court: 9 g d'un hydroxycarbonate de cérium de granulométrie inférieure à 1 µm mélangés à 0,31 g de carbonate de sodium sont chauffés à une température de 800°C pendant 15 mn en présence d'un mélange d'argon, de sulfure d'hydrogène et de sulfure de carbone à respectivement 50, 20 et 30% en volume. Les coordonnées chromatiques sont les suivantes:
L*= 51,3
a* = 50
b* = 42,6
D₅₀ = 1,5µm

Le produit se présente sous forme de grains monocristallins entiers de taille inférieure au micron.

### EXEMPLE 8

Cet exemple concerne la préparation d'un sulfure mixte de cérium et de lanthane. Un hydroxycarbonate de cérium et de lanthane ayant un rapport molaire Ce/La de 3 et de taille inférieure au micron est sulfuré à 800°C avec un palier de 30 minutes en présence de carbonate de sodium à raison de 20% molaire par rapport aux terres rares. Le gaz sulfurant est un mélange de sulfure de carbone, d'hydrogène sulfuré et d'argon dans les proportions respectives de 30, 20 et 50% en volume. Les caractéristiques couleur et granulométriques sont les suivantes:
L*= 56,3
a* = 50,7
b* = 48,9
D₅₀ = 1,7µm

Après désagglomération, le sesquisulfure mixte présente une taille moyenne de 0,85µm. Le produit se présente sous forme de grains monocristallins entiers de taille inférieure au micron.

### EXEMPLE 9

Un sulfure de praséodyme est préparé par action d'un mélange gazeux contenant 30% en volume de sulfure de carbone, 15% de sulfure d'hydrogène, le complément à 100 étant apporté par de l'argon, sur un mélange de carbonate de praséodyme et de carbonate de sodium (rapport Na/Pr de 0,2). La température de synthèse est de 800°C et le palier de 5 heures. Le sesquisulfure obtenu présente une granulométrie inférieure au micron, les coordonnées chromatiques sont les suivantes:
L* = 86,9
a* = -15,2
b* = 57,8

Le produit se présente sous forme de grains monocristallins entiers de taille inférieure au micron.

## Revendications

1. Composition à base d'un sulfure de terre rare comprenant au moins un élément alcalin caractérisée en ce que le sulfure est constitué de grains monocristallins entiers de taille moyenne d'au plus 1,5µm.

2. Composition à base de sulfure de terre rare comprenant au moins un métal alcalin, caractérisée en ce que le sulfure se présente sous forme d'agglomérats de grains agglomérés qui peuvent donner par désagglomération des grains monocristallins entiers de taille moyenne d'au plus 1,5µm.

3. Composition selon l'une des revendications précédentes, caractérisée en ce que l'élément alcalin est inclus au moins en partie dans le réseau cristallin dudit sulfure.

4. Composition selon l'une des revendications précédentes, caractérisée en ce que le sulfure de terre rare est un sesquisulfure.

5. Composition selon l'une des revendications précédentes, caractérisée en ce que la quantité d'élément alcalin est d'au plus 50% de la quantité molaire en terre rare et en ce qu'elle est notamment comprise entre 5 et 50% de cette quantité.

6. Composition selon l'une des revendications précédentes, caractérisée en ce que le sulfure présente une structure cristallographique du type Th₃P₄.

7. Composition selon l'une des revendications précédentes, caractérisée en ce que l'élément alcalin est le sodium.

8. Composition selon l'une des revendications précédentes, caractérisée en ce que le sulfure est le sesquisulfure de cérium Ce₂S₃ γ cubique.

9. Composition selon l'une des revendications 1 à 7, caractérisée en ce que le sulfure est le sesquisulfure de lanthane La₂S₃ cubique.

10. Procédé de préparation d'une composition à base d'un sulfure de terre rare comprenant au moins un élément alcalin, caractérisé en ce qu'on met en présence au moins un carbonate ou un hydroxycarbonate de terre rare avec au moins un composé d'un élément alcalin et on les chauffe en présence d'au moins un gaz choisi parmi le sulfure d'hydrogène ou le sulfure de carbone.

11. Procédé selon la revendication 10, caractérisé en ce qu'on utilise un mélange de sulfure d'hydrogène et de sulfure de carbone.

12. Procédé selon l'une des revendications 10 ou 11, caractérisé en ce qu'on utilise un carbonate comme composé d'un élément alcalin.

13. Procédé selon l'une des revendications 10 à 12, caractérisé en ce qu'on utilise un carbonate ou un hydroxycarbonate de terre rare préalablement imprégné d'un élément alcalin.

14. Procédé selon l'une des revendications 10 à 13, caractérisé en ce qu'on utilise un réacteur ouvert.

15. Procédé selon l'une des revendications 10 à 14, caractérisé en ce que la pression partielle du sulfure d'hydrogène et/ou du sulfure de carbone est comprise entre 0,1 et 1.10⁵Pa.

16. Pigment coloré, caractérisé en ce qu'il comprend au moins une composition à base d'un sulfure de terre rare selon l'une des revendications 1 à 9 ou une composition à base d'un sulfure de terre rare obtenue par le procédé selon l'une des revendications 10 à 15.

17. Utilisation d'une composition ou d'un pigment coloré selon l'une quelconque des revendications 1 à 9 et 16 dans des matières plastiques, des peintures, des lasures, des caoutchoucs, des céramiques, des glaçures, des papiers, des encres, des produits cosmétiques, des teintures et des revêtements stratifiés.

18. Compositions de matière colorées notamment du type plastiques, peintures, lasures, caoutchoucs, céramiques, glaçures, papiers, encres, produits cosmétiques, teintures et revêtements stratifiés, caractérisées en ce qu'elles comprennent une composition ou un pigment coloré tels que définis à l'une quelconque des revendications 1 à 9 et 16.

## Claims

1. Composition based on a rare-earth metal sulphide comprising at least one alkali metal element, characterized in that the sulphide comprises whole monocrystalline grains with a mean size of at most 1.5 µm.

2. Composition based on a rare-earth metal sulphide comprising at least one alkali metal, characterized in that the sulphide is provided in the form of agglomerates of agglomerated grains which can give, by deagglomeration, whole monocrystalline grains with a mean size of at most 1.5 µm.

3. Composition according to one of the preceding claims, characterized in that the alkali metal element is at least partly enclosed within the crystalline lattice of the said sulphide.

4. Composition according to one of the preceding claims, characterized in that the rare-earth metal sulphide is a sesquisulphide.

5. Composition according to one of the preceding claims, characterized in that the amount of alkali metal element is at most 50% of the molar amount of rare-earth metal and in that it is especially between 5 and 50% of this amount.

6. Composition according to one of the preceding claims, characterized in that the sulphide has a crystallographic structure of the Th₃P₄ type.

7. Composition according to one of the preceding claims, characterized in that the alkali metal element is sodium.

8. Composition according to one of the preceding claims, characterized in that the sulphide is γ cubic cerium sesquisulphide Ce₂S₃.

9. Composition according to one of claims 1 to 7, characterized in that the sulphide is cubic lanthanum sesquisulphide La₂S₃.

10. Process for the preparation of a composition based on a rare-earth metal sulphide comprising at least one alkali metal element, characterized in that at least one rare-earth metal carbonate or hydroxycarbonate is brought together with at least one compound of an alkali metal element and they are heated in the presence of at least one gas chosen from hydrogen sulphide or carbon disulphide.

11. Process according to claim 10, characterized in that a mixture of hydrogen sulphide and carbon disulphide is used.

12. Process according to either of claims 10 and 11, characterized in that a carbonate is used as compound of an alkali metal element.

13. Process according to one of claims 10 to 12, characterized in that a rare-earth metal carbonate or hydroxycarbonate impregnated beforehand with an alkali metal element is used.

14. Process according to one of claims 10 to 13, characterized in that an open reactor is used.

15. Process according to one of claims 10 to 14, characterized in that the hydrogen sulphide and/or carbon disulphide partial pressure is between 0.1 and 1 × 10⁵ Pa.

16. Coloured pigment, characterized in that it comprises at least one composition based on a rare-earth metal sulphide according to one of claims 1 to 9 or a composition based on a rare-earth metal sulphide obtained by the process according to one of claims 10 to 15.

17. Use of a composition or coloured pigment according to any one of claims 1 to 9 and 16 in plastics, paints, varnishes, rubbers, ceramics, glazings, papers, inks, cosmetic products, dyes and laminated coatings.

18. Compositions containing coloured materials, especially of the plastic, paint, varnish, rubber, ceramic, glazing, paper, ink, cosmetic product, dye and laminated coating type, characterized in that they comprise a composition or a coloured pigment as defined in any one of claims 1 to 9 and 16.

## Patentansprüche

1. Zusammensetzung auf der Basis eines Sulfids einer seltenen Erde, umfassend mindestens ein Alkalimetallelement, dadurch **gekennzeichnet**, daß das Sulfid aus ganzen monokristallinen Körnchen mit einer mittleren Größe von höchstens 1,5 µm besteht.

2. Zusammensetzung auf der Basis eines Sulfids einer seltenen Erde, umfassend mindestens ein Alkalimetallelement, dadurch **gekennzeichnet**, daß das Sulfid in Form von Agglomeraten von agglomerierten Körnchen vorliegt, die durch Desagglomeration ganze monokristalline Körnchen mit einer mittleren Größe von höchstens 1,5 µm ergeben können.

3. Zusammensetzung nach einem der vorstehenden Ansprüche, dadurch **gekennzeichnet**, daß das Alkalimetallelement mindestens teilweise in dem kristallinen Gitter des Sulfids eingeschlossen ist.

4. Zusammensetzung nach einem der vorstehenden Ansprüche, dadurch **gekennzeichnet**, daß das Sulfid der seltenen Erde ein Sesquisulfid ist.

5. Zusammensetzung nach einem der vorstehenden Ansprüche, dadurch **gekennzeichnet**, daß die Menge des Alkalimetallelements höchstens 50% der Molmenge der seltenen Erde beträgt, und daß sie insbesondere zwischen 5 und 50% dieser Menge beträgt.

6. Zusammensetzung nach einem der vorstehenden Ansprüche, dadurch **gekennzeichnet**, daß das Sulfid eine kristallographische Struktur vom Typ Th₃P₄ besitzt.

7. Zusammensetzung nach einem der vorstehenden Ansprüche, dadurch **gekennzeichnet**, daß das Alkalimetallelement Natrium ist.

8. Zusammensetzung nach einem der vorstehenden Ansprüche, dadurch **gekennzeichnet**, daß das Sulfid das Sesquisulfid von kubischem Cer γ Ce₂S₃ ist.

9. Zusammensetzung nach einem der vorstehenden Ansprüche, dadurch **gekennzeichnet**, daß das Sulfid das Sesquisulfid von kubischem Lanthan La₂S₃ ist.

10. Verfahren zur Herstellung einer Zusammensetzung auf der Basis eines Sulfids einer seltenen Erde, umfassend mindestens ein Alkalimetallelement, dadurch **gekennzeichnet**, daß man mindestens ein Carbonat oder Hydroxycarbonat einer seltenen Erde mit mindestens einer Verbindung eines Alkalimetallelements in Kontakt bringt und sie in Gegenwart mindestens eines Gases, ausgewählt aus Schwefelwasserstoff oder Schwefelkohlenstoff erhitzt.

11. Verfahren nach Anspruch 10, dadurch **gekennzeichnet**, daß man ein Gemisch aus Schwefelwasserstoff und Schwefelkohlenstoff verwendet.

12. Verfahren nach einem der Ansprüche 10 oder 11, dadurch **gekennzeichnet**, daß man ein Carbonat als Verbindung eines Alkalimetallelements verwendet.

13. Verfahren nach einem der Ansprüche 10 bis 12, dadurch **gekennzeichnet**, daß man ein Carbonat oder Hydroxycarbonat einer seltenen Erde, das zuvor mit einem Alkalimetallelement imprägniert wurde, verwendet.

14. Verfahren nach einem der Ansprüche 10 bis 13, dadurch **gekennzeichnet**, daß man einen offenen Reaktor verwendet.

15. Verfahren nach einem der Ansprüche 10 bis 14, dadurch **gekennzeichnet**, daß der Partialdruck des Schwefelwasserstoffs und/oder des Schwefelkohlenstoffs zwischen 0,1 und 1.10⁵ Pa liegt.

16. Gefärbtes Pigment, dadurch **gekennzeichnet**, daß es mindestens eine Zusammensetzung auf der Basis eines Sulfids einer seltenen Erde nach einem der Ansprüche 1 bis 9, oder eine Zusammensetzung auf der Basis eines Sulfids einer seltenen Erde, erhalten nach einem der Ansprüche 10 bis 15, umfaßt.

17. Verwendung einer Zusammensetzung oder eines gefärbten Pigments nach einem der Ansprüche 1 bis 9 und 16 in Kunststoffen, Anstrichen, Lasuren, Kautschuken, Keramiken, Glasuren, Papieren, Tinten, kosmetischen Produkten, Färbemitteln und zusammengesetzten Überzügen.

18. Zusammensetzung aus gefärbten Stoffen, insbesondere des Typs Kunststoffe, Anstriche, Lasuren, Kautschuke, Keramiken, Glasuren, Papiere, Tinten, kosmetische Produkte, Färbemittel und zusammengesetzte Überzüge, dadurch **gekennzeichnet**, daß sie eine Zusammensetzung oder ein gefärbtes Pigment wie in einem der Ansprüche 1 bis 9 und 16 definiert, umfassen.
